# EUROPÄISCHE PATENTSCHRIFT

(11) **EP 1 043 957 B1**
(45) Veröffentlichungstag und Bekanntmachung des Hinweises auf die Patenterteilung: **21.12.2005**
(21) Anmeldenummer: 99950768.4
(22) Anmeldetag: 21.10.1999
(51) Int. Cl.: A61B 18/14

(54) **MEDIZINISCHES INSTRUMENT ZUM PRÄPARIEREN VON GEWEBE**
MEDICAL INSTRUMENT FOR PREPARING TISSUE
INSTRUMENT MEDICAL POUR PREPARER DES TISSUS

(30) Priorität: 30.10.1998 DE 19850068
(43) Veröffentlichungstag der Anmeldung: 18.10.2000
(73) Patentinhaber: Karl Storz GmbH & Co. KG, 78532 Tuttlingen (DE)
(72) Erfinder: EITENMÜLLER, Jürgen, P., D-44575 Castrop-Rauxel (DE)
(74) Vertreter: Heuckeroth, Volker
(86) Internationale Anmeldenummer: PCT/EP1999/008008
(87) Internationale Veröffentlichungsnummer: WO 2000/025691

(56) Entgegenhaltungen:
- EP-A- 0 400 288
- WO-A-93/04635
- WO-A-96/22056
- DE-A- 2 415 263

## Beschreibung

Die Erfindung betrifft ein medizinisches Instrument zum Präparieren von Gewebe im menschlichen oder tierischen Körper, mit einem langerstreckten Schaft, mit zwei relativ zueinander beweglichen Maulteilen am distalen Ende des Schafts, die schneidend und/oder fassend zusammenwirken, wobei zumindest eines der Maulteile als mit Hochfrequenzstrom beaufschlagbare Elektrode ausgebildet ist.

Ein derartiges Instrument zum Präparieren von Gewebe im menschlichen oder tierischen Körper ist aus dem Dokument WO-A-96/22056 bekannt.

Unter Präparieren wird im Sinne der vorliegenden Erfindung Schneiden von Gewebe und/oder Fassen von Gewebe verstanden, um Gewebe im Körper abzutrennen und zu entfernen oder beiseite zu legen.

Ein derartiges Instrument wird vorzugsweise in der minimal-invasiven Chirurgie verwendet, bei der im Unterschied zu der herkömmlichen offenen Chirurgie das Instrument durch eine kleine Inzision von außen in das Operationsgebiet geführt wird, wobei der Operationsvorgang unter endoskopischer Kontrolle durchgeführt wird.

Bei solchen Instrument wird häufig nicht nur die rein mechanisch schneidende Wirkung der Maulteile zum Ab- oder Durchtrennen von Gewebe genutzt, sondern die Maulteile oder zumindest eines der Maulteile werden zusätzlich mit monopolarem Hochfrequenzstrom beaufschlagt, um einerseits die Schneidwirkung durch die thermische Wirkung des Hochfrequenzstroms in dem Gewebe zu erhöhen, und um andererseits durch die Wärmeentwicklung eine Koagulation des Gewebes an der Trennungsstelle herbeizuführen, um die beim Durchtrennen des Gewebes auftretende Blutung zu vermindern oder gar zu stillen.

Während in der minimal-invasiven Chirurgie anfangs nur das Präparieren von kleineren Gewebeteilen erfolgreich durchgeführt werden konnte, geht die Entwicklung der minimal-invasiven Chirurgie nunmehr dahin, auch größere Gewebeteile, bspw. den Dickdarm, oder Organe zu entfernen. Bei der Entfernung größerer Gewebeteile ist es erforderlich, auch größere Gewebebrücken zu durchtrennen, die darüber hinaus größere Gefäße enthalten können. Die dabei möglicherweise auftretenden stärkeren Blutungen können jedoch mit dem monopolar betriebenen Instrument nicht beherrscht werden, so daß die Durchtrennung von größeren Gewebebrücken zusätzlich den Einsatz von bipolar betriebenen Koagulationsinstrumenten erfordert.

Dies bedeutet jedoch, daß der Chirurg beim Präparieren, d.h. zum Trennen solcher größeren Gewebebrücken, mehrmals einen Instrumentenwechsel durchführen muß, wenn er sich nicht allein auf die geringere koagulierende Wirkung des monopolar betriebenen Instruments verlassen will. Die Operation verläuft demnach so, daß der Chirurg hauptsächlich mit dem monopolar betriebenen Instrument Gewebe entfernt und, wenn er an eine ein größeres Gefäß enthaltende Gewebebrücke gelangt, das monopolar betriebene schneidende Instrument aus dem Körper des Patienten entnehmen und ein bipolar betriebenes Koagulationsinstrument an die Operationsstelle führen muß. Dieser Instrumentenwechsel ist jedoch umständlich, verlängert die Operationsdauer unter Umständen erheblich und erhöht die Gefahr von Komplikationen.

In dem eingangs genannten Dokument WO-A-96/22056 ist ein endoskopisches Instrument beschrieben, das einen langerstreckten Schaft aufweist, an dessen distalem Ende zwei relativ zueinander bewegliche Maulteile angeordnet sind, die fassend zusammenwirken. Auf den Innenseiten der Maulteile ist ein Schlitz in Längsrichtung der Maulteile vorhanden, in den von proximal nach distal eine Klinge vorgeschoben werden kann, um zwischen den Maulteilen gefaßtes Gewebe zu durchtrennen. Die Klinge kann dabei auch als leitendes Element ausgebildet sein, um die Klinge mit Hochfrequenzstrom beaufschlagen zu können. Des weiteren können auch die Maulteile mit Hochfrequenzstrom beaufschlagt werden.

Aus dem DE-Firmenkatalog der Firma Karl Storz GmbH & Co., Tuttlingen, "Karl Storz-Endoskope", Band Gynäkologie 2/96, Seite BI/COA 5/7, ist nun ein bipolares Koagulationsinstrument bekannt, das am distalen Ende zwei voneinander beabstandete Paare von Maulteilen aufweist, von denen jedes Paar als mit Hochfrequenzstrom beaufschlagbare Elektrode ausgebildet ist. Die beiden Maulteilpaare sind voneinander beabstandet und können daher als bipolare Elektrodenanordnung betrieben werden. Zwischen den Maulteilpaaren ist ein Schneidwerkzeug in Form eines Skalpells angeordnet, das wahlweise vor- und zurückbewegbar ist. Mit diesem Instrument kann zwischen die beiden Maulteilpaare ein Gefäß geklemmt und koaguliert und anschließend durch Voranschieben des Schneidwerkzeuges durchtrennt werden. Dieses Instrument ist jedoch weniger geeignet, hauptsächlich als Präparierinstrument zum Durchtrennen von Gewebe verwendet zu werden, sondern die Hauptfunktion dieses Instruments besteht in der bipolaren Koagulation.

Dazu ähnliche Instrumente sind in der WO 95/15124 und der US-A-5 445 638 beschrieben.

Bei den zuvor genannten bekannten Instrumenten ist die Schneidwirkung mittels des zwischen dem Koagulationselektroden angeordneten verschiebbaren Skalpells rein mechanisch, d.h. ohne Unterstützung durch Hochfrequenzstrom. Weiterhin eignen sich diese Schneideinrichtungen vorrangig nur zum Durchtrennen von Gefäßen, nicht jedoch zum Einbringen von längeren Schnitten und auch nicht zum Fassen.

Für den eingangs genannten Zweck des Präparierens von Gewebe im menschlichen oder tierischen Körper, bei dem größere Gewebeteile entfernt und dazu größere Gewebebrücken durchtrennt werden müssen, ist daher ein Instrument der eingangs genannten Art funktionsgerecht, da bei einem solchen Instrument das Durchtrennen von Gewebe im Vordergrund steht.

Der Erfindung liegt daher die Aufgabe zugrunde, ein Instrument der eingangs genannten Art dahingehend weiterzubilden, daß mit diesem Instrument auch größere Gewebeteile und damit größere, größere Gefäße enthaltende Gewebebrücken durchtrennt werden können, ohne daß die Gefahr einer übermäßigen Blutung besteht.

Erfindungsgemäß wird diese Aufgabe hinsichtlich des eingangs genannten Instruments dadurch gelöst, daß eine weitere mit Hochfrequenzstrom beaufschlagbare Elektrode vorgesehen ist, die wahlweise aus einer zurückgezogenen proximalen Position in eine distale, den Maulteilen seitlich benachbarte Position verschiebbar ist, in der sie im Zusammenwirken mit dem zumindest einen als Elektrode ausgebildeten Maulteil den zweiten Pol einer Elektrodenanordnung zum Fassen und bipolaren Koagulieren von Gewebe zwischen der weiteren Elektrode und den Maulteilen bildet.

Es wird demnach erfindungsgemäß ein Instrument bereitgestellt, mit dem es möglich ist, unter standardmäßiger Verwendung eines monopolar betriebenen Instruments Gewebe durch Schneiden und/oder Fassen zu präparieren und eventuell sehr kleine Gefäße monopolar wie herkömmlich zu koagulieren.

Gelangt der Chirurg dabei jedoch an eine Gewebebrücke, die größere Gefäße zu beinhalten scheint, so kann diese Gewebebrücke durch Vorschieben der weiteren Elektrode in die distale, den Maulteilen benachbarte Position zwischen den Maulteilen und der weiteren Elektrode gegriffen werden. Das zumindest eine als Elektrode ausgebildete Maulteil und die weitere Elektrode können dann mit bipolarem Hochfrequenzstrom beaufschlagt werden, so daß das dazwischen liegende Gewebe bipolar koaguliert werden kann. Mit anderen Worten bilden die Maulteile den einen Elektrodenpol und die weitere Elektrode den zweiten Elektrodenpol. Anschließend kann dann, vorzugsweise nachdem die weitere Elektrode wieder in ihre proximale Position zurückgeschoben wurde, mit den Maulteilen weiter präpariert werden, entweder rein mechanisch oder monopolar strombeaufschlagt. Die Erfindung stellt somit ein monopolar betreibbares Instrument zum Präparieren von Gewebe in Kombination mit einer wahlweise zuschaltbaren bipolaren Koagulationseinrichtung bereit, wodurch der erhebliche Vorteil erreicht wird, daß zum Präparieren und zum bipolaren Koagulieren kein Instrumentenwechsel erforderlich ist und auch größere Gewebeteile unter Vermeidung von Blutungen schnell und sicher präpariert werden können.

Somit wird die der Erfindung zugrundeliegende Aufgabe vollkommen gelöst.

In einer bevorzugten Ausgestaltung sind die Maulteile aus der Längsachse des Schafts heraus gekrümmt und ist die weitere Elektrode auf der konkaven Seite der Maulteile angeordnet.

Diese Maßnahme hat den Vorteil, daß beim Vorschieben der weiteren Elektrode in ihre distale Position das bipolar zu koagulierende Gewebe zwischen den Maulteilen und der weiteren Elektrode sicher gegriffen und gehalten werden kann. Die gekrümmte Ausgestaltung der Maulteile wirkt dabei als Fanghaken und Widerlager beim Vorschieben der weiteren Elektrode gegen ein seitliches Ausweichen des Gewebes.

In einer weitere bevorzugten Ausgestaltung ist die weitere Elektrode flächig ausgebildet und weist etwa die gleiche Breitenabmessung auf wie die beiden Maulteile.

Hierbei ist von Vorteil, daß bereits mit einem Koaguliervorgang größere Gewebepartien koaguliert werden können. Selbstverständlich ist es auch möglich, unter seitlichem Versatz des Instrumentes mehrmals zu koagulieren, wenn eine besonders große Gewebebrücke durchtrennt werden muß, die besonders große Gefäße beinhaltet. Die Maulteile können beim bipolaren Koagulieren des Gewebes geöffnet oder geschlossen sein, wobei in der geöffneten Stellung der Maulteile die wirksame Elektrodenfläche der Maulteile noch vergrößert ist.

In einer weiteren bevorzugten Ausgestaltung bildet die weitere Elektrode mit zumindest einem der Maulteile einen Fangraum zum Greifen von Gewebe.

Diese Ausgestaltung hat zum Vorteil, daß zwischen der weiteren Elektrode und den Maulteilen noch dickeres Gewebe und noch dikkere Gefäße gegriffen werden können.

In einer weiteren bevorzugten Ausgestaltung ist die weitere Elektrode in ihre zurückgezogene Position im Schaft versenkt aufgenommen. Hierbei ist von Vorteil, daß die weitere Elektrode in ihrer zurückgezogenen Position das vorrangig mit dem Instrument durchzuführende Präparieren von Gewebe mittels der beiden Maulteile nicht behindert und nur im Bedarfsfall aus dem Schaft herausgeschoben wird.

In einer weiteren bevorzugten Ausgestaltung ist die weitere Elektrode am distalen Ende auf der den Maulteilen zugewandten Seite abgeschrägt.

Hierbei ist von Vorteil, daß beim Vorschieben der weiteren Elektrode vermieden wird, daß das zwischen den Maulteilen und der weiteren Elektrode zu fassende Gewebe von der weiteren Elektrode verdrängt wird und dann nicht zwischen der weiteren Elektrode und den Maulteilen koaguliert werden kann. Auch ist die zuvor erwähnte gekrümmte Ausgestaltung der Maulteile in diesem Zusammenhang besonders vorteilhaft, da die konkave Seite der Maulteile in der Art eines Fanghakens und Widerlagers beim Vorschieben der weiteren Elektrode wirkt, so daß das Gewebe beim Vorschieben der weiteren Elektrode nicht seitlich ausweichen kann.

In einer weiteren bevorzugten Ausgestaltung ist die weitere Elektrode zumindest teilweise elastisch ausgebildet und beschreibt beim Vorschieben eine Bahnkurve, die anfangs etwa in Längsrichtung des Schafts mit oder ohne geringfügig von den Maulteilen abgewandter Komponente und zu der distalen Position hin mit zu den Maulteilen hin gerichteter Komponente verläuft.

Bei der elastischen Ausgestaltung der Elektrode ist von Vorteil, daß die Elektrode beim Vorschieben durch eine geeignete Führung oder eine der Elektrode verliehene Vorspannung beim Vorschieben eine gekrümmte Bahnkurve beschreibt, wodurch das zu koagulierende Gewebe beim Vorschieben leichter umgriffen und in der maximal vorgeschobenen distalen Position zwischen den Maulteilen und der weiteren Elektrode sicher gehalten bzw. eingeklemmt werden kann.

Dabei ist es bei einem Ausführungsbeispiel bevorzugt, wenn am Schaft eine Anlaufschräge angeordnet ist, auf die eine an der weiteren Elektrode ausgebildete Anlaufschräge beim Vorschieben der Elektrode aufläuft, wodurch die Elektrode in der distalen Position gegen die Maulteile gedrückt wird.

Hierbei wird vorteilhafterweise erreicht, daß das Gewebe zwischen der maximal vorgeschobenen weiteren Elektrode und den Maulteilen mit höherer Klemmkraft gehalten werden kann.

In einer weiteren bevorzugten Ausführungsbeispiel ist am Schaft eine Anlaufschräge angeordnet, so daß beim Vorschieben der weiteren Elektrode diese von den Maulteilen zunächst abspreizt wird und in der distalen Position elastisch gegen die Maulteile federt.

Bei dieser Ausgestaltung ist von Vorteil, daß die weitere Elektrode beim Vorschieben zunächst von den Maulteilen weggerichtet vorgeschoben werden kann, wodurch wiederum noch größere Gewebeteile sicher umgriffen werden können. Durch das elastische Federn der weiteren Elektrode in ihrer distalen Position auf die beiden Maulteile zu wird dann das umschlossene Gewebe automatisch sicher zwischen den Maulteilen und der weitere Elektrode zum bipolaren Koagulieren festgehalten.

In einer weiteren bevorzugten Ausgestaltung ist am proximalen Ende des Instruments eine Handhabe angeordnet, die zwei Griffteile zur Betätigung der Maulteile und ein weiteres Griffteil zur Betätigung der weiteren Elektrode aufweist, wobei die Griffteile eine mit einer Hand bedienbare Griffanordnung bilden.

Diese Maßnahme hat den Vorteil, daß die Betätigung der Maulteile einerseits und die wahlweise Betätigung der weiteren Elektrode, nämlich das Vorschieben und Zurückschieben der weiteren Elektrode, besonders bequem und vor allem in Einhandbedienung durchgeführt werden kann. Auf diese Weise ist die Handhabung des erfindungsgemäßen Instruments verbessert und ermöglicht ein ermüdungsfreies Arbeiten mit dem Instrument.

Dabei ist es bevorzugt, wenn das weitere Griffteil über eine Hebelanordnung mit der weiteren Elektrode derart verbunden ist, daß durch Ziehen des weiteren Griffteils die weitere Elektrode aus ihrer proximalen in ihre distale Position geschoben wird.

Diese Ausgestaltung des weiteren Griffteils verbessert weiterhin die Bedienungsfreundlichkeit des Instrumentes, weil eine Betätigung des weiteren Griffteils zum Vorschieben der weiteren Elektrode durch Zug eine bessere Kraftdosierung und somit ein sicheres Arbeiten mit dem Instrument ermöglicht.

In einer weiteren bevorzugten Ausgestaltung ist die weitere Elektrode in ihre proximale Position vorgespannt, in die sie nach Loslassen des weiteren Griffteils selbsttätig zurückkehrt.

Durch diese Maßnahme wird die Bedienungsfreundlichkeit des erfindungsgemäßen Instruments noch weiter verbessert.

In einer weiteren bevorzugten Ausgestaltung ist die weitere Elektrode über ein axial beweglich in dem Schaft aufgenommenes Betätigungselement mit dem weiteren Griffteil verbunden, wobei das Betätigungselement weiterhin über einen Schleifkontakt mit einer Hochfrequenzstrom-Zuführung verbunden ist.

Hierbei ergibt sich durch das axial bewegliche Betätigungselement eine besonders vorteilhafte Kraftübertragung zwischen dem weiteren Griffteil und der weiteren Elektrode einerseits und andererseits durch den Schleifkontakt eine vorteilhaft konstruktiv einfache und sichere Beaufschlagung der weiteren Elektrode durch Stromleitung durch das bewegliche Betätigungselement.

In einer bevorzugten Ausgestaltung läßt der Schleifkontakt eine Stromdurchführung auf die weitere Elektrode erst zu, wenn die Elektrode in die distale Position oder nahezu in die distale Position vorgeschoben ist.

Durch diese Ausgestaltung wird die Betriebssicherheit des erfindungsgemäßen Instruments vorteilhaft verbessert.

In einer weiteren bevorzugten Ausgestaltung ist das Betätigungselement mit dem weiteren Griffteil abnehmbar, vorzugsweise durch Verrasten verbunden.

Hierbei ist von Vorteil, daß eine leichte Zerlegbarkeit des Instruments in bezug auf die erfindungsgemäß vorgesehene Koagulationseinrichtung ermöglicht wird.

In einer weiteren bevorzugten Ausgestaltung sind die Maulteile über ein axial beweglich in dem Schaft aufgenommenes Betätigungselement abnehmbar mit den Griffteilen verbunden.

Durch diese Maßnahme wird die Zerlegbarkeit des erfindungsgemäßen Instruments noch verbessert, so daß sich das erfindungsgemäße Instrument leicht reinigen läßt und somit den hohen Hygieneanforderungen hinsichtlich Sterilität vollends genüge leistet.

Dabei ist bevorzugt, wenn die Maulteile über einen Bajonettverschluß dem distalen Ende des Schafts verbunden sind.

Durch diese Ausgestaltung der Verbindung der Maulteile mit dem Schaft wird eine zum Verbinden und Lösen der Maulteile von dem Schaft vorteilhaft einfach bedienbare Ausgestaltung geschaffen.

Besonders bevorzugt ist es, wenn das Instrument in die Baugruppen aus den Maulteilen mit dem mit diesen verbundenen Betätigungselement, der weiteren Elektrode mit dem weiteren Betätigungselement, der Handhabe und dem Schaft zerlegbar ist.

Durch diese Zerlegbarkeit des Instrumentes in die vier vorstehend genannten Baugruppen läßt sich das erfindungsgemäße Instrument besonders gründlich reinigen und genügt somit höchsten Hygieneanforderungen.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in ihrer jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine Gesamtseitenansicht eines medizinischen Instruments zum Präparieren von Gewebe;
- Fig. 2: einen Längsschnitt durch das distale Ende des Instruments in Fig. 1 in einer gegenüber Fig. 1 um 90° verdrehten Ansicht (Draufsicht), wobei die weitere Elektrode in ihrer proximalen Position dargestellt ist;
- Fig. 3: eine der Fig. 2 entsprechende Darstellung mit nach distal vorgeschobener weiterer Elektrode;
- Fig. 4: eine Draufsicht auf einen Handhabungsbereich des Instrumentes in Fig. 1, teilweise im Schnitt;
- Fig. 5: die Handhabe des Instruments in einer Seitenansicht in Alleinstellung;
- Fig. 6: einen Teil der Handhabe in Fig. 5, die das weitere Griffteil zur Betätigung der weiteren Elektrode in Alleinstellung zeigt;
- Fig. 7: einen Schnitt entlang der Linie VII-VII in Fig. 6, wobei das weitere Griffteil in aufgeklappter Position dargestellt ist;
- Fig. 8: das distale Ende eines weiteren Ausführungsbeispiels eines Instruments in einer Fig. 2 entsprechenden Schnittdarstellung;
- Fig. 9: das distale Ende in Fig. 8 in einer Fig. 3 entsprechenden Darstellung, in der die weitere Elektrode in ihre distale Position vorgeschoben ist;
- Fig. 10: das distale Ende eines noch weiteren Ausführungsbeispiels eines Instruments in einer Fig. 8 entsprechenden Darstellung; und
- Fig. 11: das distale Ende in Fig. 10 in einer Fig. 9 entsprechenden Darstellung, in der die weitere Elektrode in ihre distale Position vorgeschoben ist.

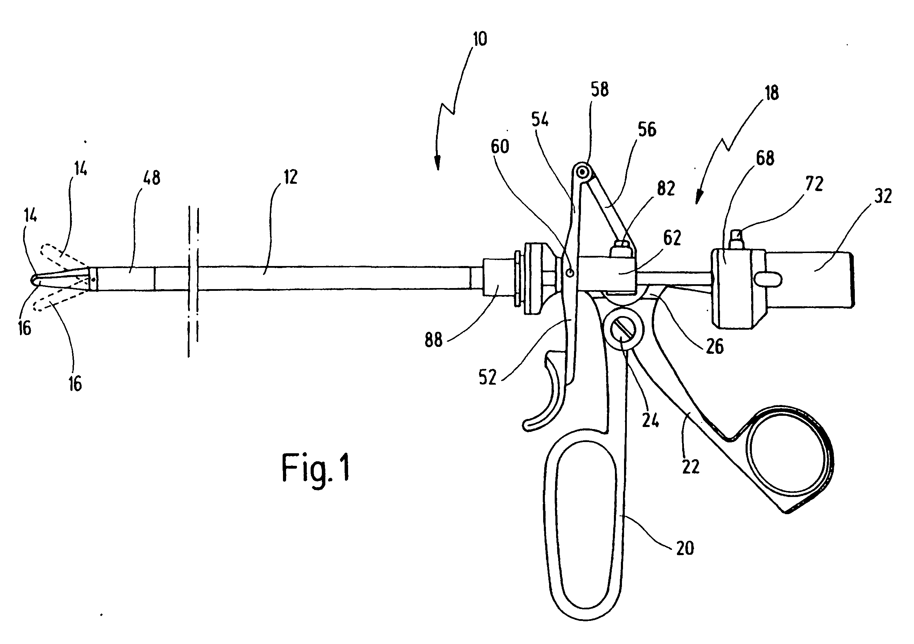

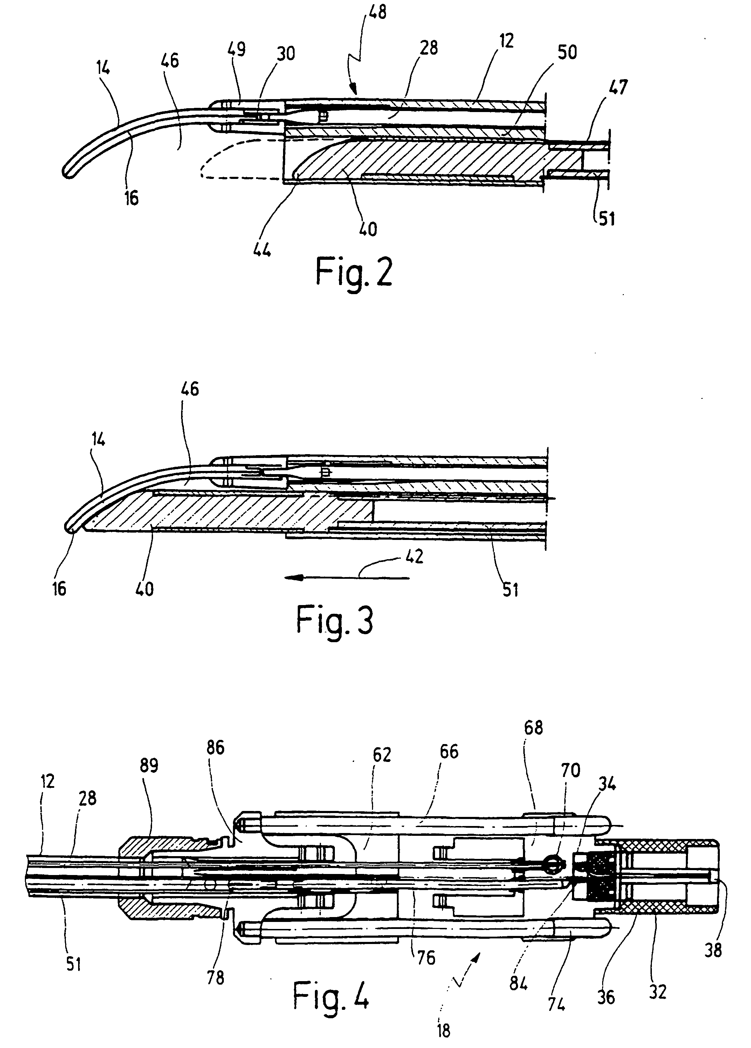

In Fig. 1 ist ein mit dem allgemeinen Bezugszeichen 10 versehenes medizinisches Instrument zum Präparieren von Gewebe im menschlichen oder tierischen Körper dargestellt. Teile des Instruments 10 sind in den weiteren Figuren 2 bis 7 im Detail dargestellt.

Das Instrument 10 dient in der minimalinvasiven Chirurgie zum Schneiden und Heraustrennen von Gewebe und eignet sich insbesondere zum Präparieren von großen Gewebeteilen, bei deren Präparation größere Gewebebrücken, die auch größere Gefäße enthalten können, durchtrennt werden müssen.

Das Instrument 10 weist einen langerstreckten Schaft 12 auf. Der Schaft 12 ist im wesentlichen als zylindrisches Rohr ausgebildet.

Am distalen Ende des Schafts 12 sind zwei relativ zueinander bewegliche Maulteile 14 und 16 angeordnet, wobei in dem gezeigten Ausführungsbeispiel beide Maulteile 14 und 16 beweglich sind. Eine Ausgestaltung, bei der nur eines der Maulteile, entweder das Maulteil 14 oder das Maulteil 16, beweglich ist, ist jedoch ebenfalls möglich.

Die Maulteile 14 und 16 wirken als Schneidwerkzeug zusammen, um Gewebe durchzutrennen. Die Maulteile 14 und 16 sind in Fig. 1 mit durchgezogenen Linien in ihrer Schließlage und mit unterbrochenen Linien in ihrer Offenlage dargestellt. Die Maulteile 14 und 16 können anstelle als Schneidwerkzeuge auch als Faßwerkzeuge oder als eine Kombination von Schneid- und Faßwerkzeugen ausgebildet sein.

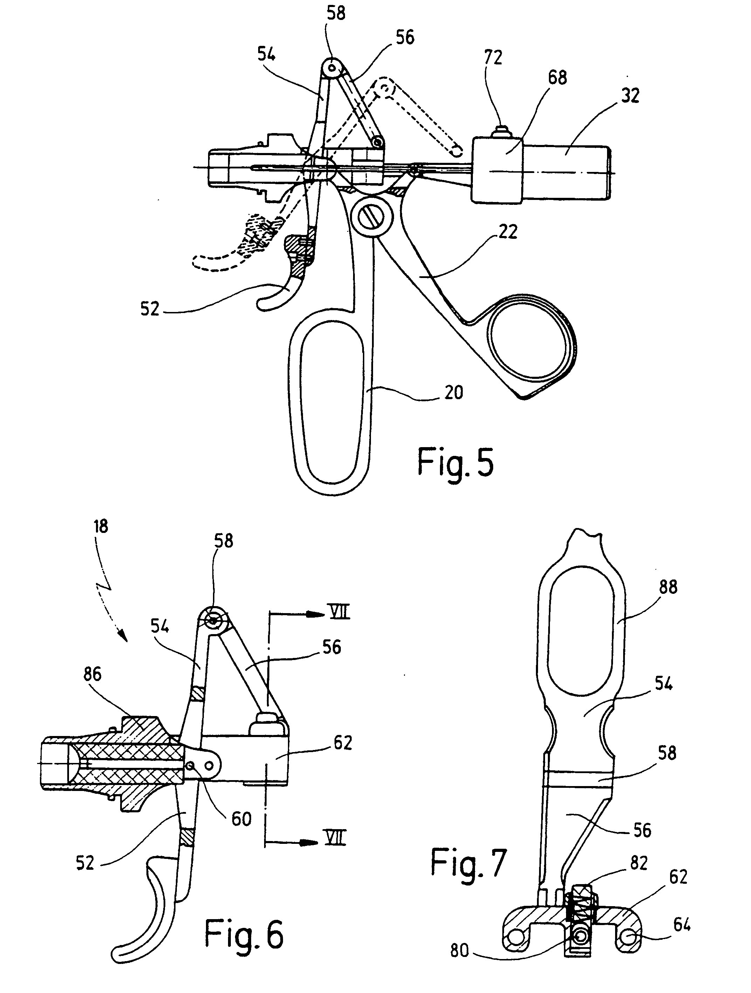

Am proximalen Ende des Schafts 12 weist das Instrument 10 eine mit dem allgemeinen Bezugszeichen 18 versehene Handhabe auf. Die Handhabe 18 ist in Fig. 5 in Alleinstellung in Seitenansicht und in Fig. 4 in teilweise geschnittener Draufsicht mit daran befestigtem Schaft 12 dargestellt, wobei von dem Schaft 12 in Fig. 4 lediglich das proximale Ende dargestellt ist.

Die Handhabe 18 weist ein erstes Griffteil 20 und ein zweites Griffteil 22 auf, wobei die Griffteile 20 und 22 der Betätigung der Maulteile 14 und 16 zum Öffnen und Schließen derselben dienen. Dabei ist das Griffteil 20 unbeweglich, d.h. schaftfest, während das Griffteil 22 beweglich ist. Die Griffteile 20 und 22 sind über ein Gelenk 24 miteinander verbunden. Ein Schenkel 26 des beweglichen Griffteils 22 ist gemäß Fig. 4 und Fig. 2 über ein in dem Schaft 12 axial verschiebbar aufgenommenes Betätigungselement 28 mit den Maulteilen 14 und 16 kraftschlüssig verbunden. Das Betätigungselement 28 arbeitet zum Öffnen der Maulteile 14 und 16 auf Druck, und durch Schließen des beweglichen Griffteils 22 in Richtung des unbeweglichen Griffteils 20 wird das Betätigungselement 28 durch den Schenkel 26 nach proximal verschoben, wodurch die Maulteile 14 und 16 geschlossen werden, wobei das Betätigungselement 28 an einer Kniehebelanordnung 30 am proximalen Ende der Maulteile 14 und 16 angreift, die die Maulteile 14 und 16 öffnet und schließt.

Ferner ist zumindest eines der Maulteile 14 und 16, bevorzugt sind jedoch beide Maulteile 14 und 16 als mit Hochfrequenzstrom beaufschlagbare Elektroden ausgebildet, wobei am proximalen Ende des Instruments 10 ein Hochfrequenzstromanschluß 32 zum Anschließen eines nicht dargestellten Hochfrequenzstromkabels vorgesehen ist. Die Stromzuführung erfolgt dabei über eine Stromleitung 34 über das Betätigungselement 28 auf eines der Maulteile 14 oder 16 oder auf beide Maulteile 14 und 16. Das Betätigungselement 28 ist dazu entsprechend gegen den Schaft 12 isoliert. Die Maulteile 14 bzw. 16 können dabei entweder mit monopolarem Hochfrequenzstrom beaufschlagt werden, um Gewebe monopolar zu schneiden, oder die Maulteile 14 bzw. 16 dienen als Gegenelektrode bzw. Gegenpol für eine bipolare Koagulation von Gewebe, wie im folgenden noch beschrieben wird.

Der Hochfrequenzstromanschluß 32 besteht aus einem Gehäuse 36 aus Kunststoff, in dem der eigentliche Kontaktfinger 38 eingetaucht aufgenommen ist.

Wie zuvor bereits erwähnt, weist das Instrument 10 nicht nur die Funktion auf, Gewebe mittels der Maulteile 14 und 16 zu durchtrennen, sondern es ist auch möglich, mit dem Instrument 10 Gewebe bipolar zu koagulieren.

Dazu weist das Instrument 10 eine weitere mit Hochfrequenzstrom beaufschlagbare Elektrode 40 auf, die in Figuren 2 und 3 zu sehen ist. In der Darstellung gemäß Fig. 1 ist die Elektrode 40 nicht sichtbar, da die weitere Elektrode 40 seitlich der Maulteile 14 und 16 angeordnet ist und von diesen in Fig. 1 verdeckt wird.

Die weitere Elektrode 40 ist in Fig. 2 in ihrer maximal proximal zurückgezogenen Position dargestellt, in der die weitere Elektrode 40 in dem Schaft 12 versenkt aufgenommen ist. In Fig. 3 ist die weitere Elektrode 40 gemäß einem Pfeil 42 in ihre distale Position verschoben, in der sie im Zusammenwirken mit dem zumindest einen als Elektrode ausgebildeten Maulteil 14 bzw. 16 bzw. mit beiden Maulteilen 14 und 16 den zweiten Pol einer Elektrodenanordnung eines bipolaren Koagulationswerkzeuges bildet, wobei das zumindest eine Maulteil 14 bzw. 16 oder beide Maulteile 14 und 16 den ersten Pol bilden.

Die weitere Elektrode 40 ist an ihrem distalen Ende 44 abgeschrägt ausgebildet.

Die Maulteile 14 und 16 sind weiterhin aus der Längsachse des Schafts 12 heraus gekrümmt ausgebildet, wobei die weitere Elektrode 40 auf der konkaven Seite der Maulteile 14 und 16 angeordnet ist. Die gekrümmt ausgebildeten Maulteile 14 und 16 dienen beim Vorschieben der weiteren Elektrode 40 dazu, das zwischen die weitere Elektrode 40 und die Maulteile 14 und 16 zu bringende Gewebe in der Art eines Fanghakens festzuhalten, wobei auch die schräge Ausgestaltung des distalen Endes 44 der weiteren Elektrode 40 dazu beiträgt, daß zwischen der weiteren Elektrode 40 und den Maulteilen 14 bzw. 16 ein Fangraum 46 gebildet wird, in dem das anschließend bipolar zu koagulierende Gewebe gefangen wird. Dies geht auch aus Fig. 3 hervor, in der der Fangraum 46 als lichter Raum zwischen der weiteren Elektrode 40 und den Maulteilen 14 und 16 selbst in der maximal nach distal verschobenen Position der weiteren Elektrode 40 verbleibt. Am proximalen Ende weist die Elektrode 40 eine Gleitschicht 47 und Isolierschicht aus Kunststoff auf.

Die weitere Elektrode 40 ist in Richtung quer zur Zeichenebene der Figuren 2 und 3 flächig ausgebildet und weist in dieser Dimension etwa die gleiche Breitenabmessung auf wie die beiden Maulteile 14 und 16.

Weiterhin ist die in Figuren 2 und 3 dargestellte Ausführungsform der weiteren Elektrode 40 starr und massiv.

Im Bereich seines distalen Endes weist der Schaft 12 eine Buchse 48 auf, die gemäß Fig. 2 eine Zwischenwand 50 aufweist, durch die das Betätigungselement 28 und die weitere Elektrode 40 voneinander getrennt sind. Im sich proximal an die Buchse 48 anschließenden Abschnitt des Schafts 12 ist der Schaft 12 ohne eine derartige Zwischenwand ausgebildet. Die Buchse 48 ist mit dem übrigen Teil des Schafts 12 fest verbunden. Die Maulteile 14, 16 und das mit diesen verbundene Betätigungselement 28 sind über einen nicht näher dargestellten Bajonettverschluß, der an einer Gabel 49 ausgebildet ist, lösbar (über eine 90°-Drehung) mit der Buchse 48 verbunden.

Zur Betätigung der weiteren Elektrode 40 ist diese über ein Betätigungselement 51 in Form eines dünnen Rohres mit einem weiteren Griffteil 52 an der Handhabe 18 verbunden. Das weitere Griffteil 52 bildet mit den Griffteilen 20 und 22 eine mit einer Hand bedienbare Griffanordnung. Das Griffteil 52 ist dabei distalseitig der Griffteile 20 und 22 angeordnet und in der Art eines Pistolenabzugs ausgestaltet, der beispielsweise bequem mit dem Zeigefinger bedient werden kann. Das Griffteil 22 kann mit dem Daumen, und das Griffteil 20 kann beispielsweise mit dem Mittelfinger oder mit dem Mittelfinger und zusätzlich dem Ringfinger bedient werden. Zum Betätigen der Maulteile 14 und 16 einerseits und zum Vor- und Zurückschieben der weiteren Elektrode 40 ist somit ein Umgreifen oder eine Veränderung der Handhaltung nicht erforderlich.

Das weitere Griffteil ist über eine Hebelanordnung aus einem ersten Hebel 54 und einem zweiten Hebel 56 mit dem Betätigungselement 51 verbunden. Der erste Hebel 54 und der zweite Hebel 56 sind über ein Gelenk 58 untereinander verbunden. Der erste Hebel 54 ist bezüglich dem Schaft 12 um einen Drehpunkt 60 verschwenkbar. Der zweite Hebel 56 ist mit seinem dem Gelenk 58 gegenüberliegenden Ende an einem Schlitten 62 befestigt, der wiederum mit dem Betätigungselement 51 lösbar verbunden ist.

In Fig. 5 ist das Griffteil 52 zur Betätigung der weiteren Elektrode 40 mit unterbrochenen Linien in einer Stellung dargestellt, in der die weitere Elektrode 40 gemäß Fig. 2 in ihrer proximalen Position in dem Schaft 12 aufgenommen ist. Durch Ziehen des Griffteils 52 in proximaler Richtung in die in Fig. 5 mit durchgezogenen Linien dargestellte Stellung bewirkt die Hebelanordnung aus den Hebeln 54 und 56 eine Bewegungsumkehr, so daß der Schlitten 62 und über das Betätigungselement 51 die weitere Elektrode 40 nach distal verschoben wird. Der Schlitten 62 gleitet dabei auf an der Handhabe 18 seitlich angeordneten Führungsstangen 66, die durch Bohrungen 64 in dem Schlitten 62 durchgehen.

Der Betätigungsweg des Schlittens 62 und damit der weiteren Elektrode 40 beträgt etwa 2,5 cm. Während der Bewegung des Schlittens 62 bleibt der Hochfrequenzstromanschluß 32 ortsfest, wenn die Maulteile 14 und 16 nicht mittels der Griffteile 20 und 22 betätigt werden.

Wie bereits zuvor beschrieben wurde, sind die Maulteile 14 und 16 über ein Betätigungselement 28 mit dem beweglichen Griffteil 22 verbunden. Der Schenkel 26 des beweglichen Griffteils 22 ist mit einem weiteren Schlitten 68 verbunden, an dem das Betätigungselement 28 mittels einer Raste 70 festgelegt ist. Am proximalen Ende des Schlittens 68 ist der Hochfrequenzstromanschluß 32 mit diesem fest verbunden. Ein Rastknopf 72 (vgl. Fig. 1) dient zum Lösen der Verrastung zwischen dem Betätigungselement 28 und dem Schlitten 68, um die Maulteile 14, 16 mit dem Betätigungselement 28 nach Lösen des Bajonettverschlusses durch Drehen der Maulteile 14, 16 um 90° nach distal aus dem Schaft 12 herausziehen zu können.

Der Schlitten 68 ist wie der Schlitten 62 auf den Führungsstangen 66 axial verschiebbar, wozu an dem Schlitten 68 Hülsen 74 befestigt sind, in die die Führungsstangen 66 eingreifen. Beim Betätigen des Griffteiles 22 wird das Betätigungselement 28 somit axial verschoben, wobei im Unterschied zu dem Betätigungsweg des Betätigungselements 52 der Betätigungsweg des Betätigungselementes 28 wesentlich geringer ist und nur etwa wenige Millimeter beträgt.

Das Betätigungselement 51 zum axialen Verschieben der weiteren Elektrode 40 ist weiterhin mit einer Hochfrequenzstrom-Zuführung 76 verbunden. Die Hochfrequenzstrom-Zuführung 76 weist einen Stab auf, der an seinem distalen Ende einen Schleifkontakt 78 aufweist. Das Betätigungselement 51 ist über den Schleifkontakt 78 hinaus auf die Hochfrequenzstrom-Zuführung 76 bis zu dem Schlitten 62 aufgeschoben, an dem das Betätigungselement 51 mit dem Schlitten 62 mittels einer Raste 80 (vgl. Fig. 7) verrastet ist. Zum Lösen der Verrastung zwischen dem Betätigungselement 51 und dem Schlitten 62 ist wiederum ein Rastknopf 82 vorgesehen, so daß nach Lösen der Raste 80 durch Herunterdrücken des Rastknopfes 82 das Betätigungselement 51 nach distal aus dem Schaft 12 herausgezogen werden kann.

Die Hochfrequenzstrom-Zuführung 76 ist über eine weitere Stromleitung 84 mit dem Kontaktfinger 38 des Hochfrequenzstromanschlusses verbunden.

Der Schleifkontakt 78 wirkt mit dem Betätigungselement 51 so zusammen, daß eine Stromübertragung von der Hochfrequenzstrom-Zuführung 76 auf das Betätigungselement 51 und damit auf die weitere Elektrode erst dann möglich wird, wenn das Betätigungselement 51 und damit die weitere Elektrode 40 beinahe ihre maximale distale Position erreicht hat. Dies wird dadurch erreicht, daß der Schleifkontakt 78, in der proximalen Position des Betätigungselementes, auf eine Isolierschicht an dem Betätigungselement 51 greift und erst dann auf Metall greift, wenn sich die Elektrode 40 nahezu in ihrer distalen Position befindet.

Gemäß Figuren 4 und 6 weist die Handhabe 18 an ihrem distalen Ende einen Gehäuseabschnitt 86 auf, an dem einerseits die Führungsstangen 66 (vgl. Fig. 4) und andererseits der erste Hebel 54 des weiteren Griffteils 52 befestigt sind. Aus Fig. 7, in der das weitere Griffteil 52 in um das Gelenk 58 aufgeklappter Stellung dargestellt ist, geht weiter hervor, daß der erste Hebel 54 des Griffteils 52 eine ringförmige Gabelung 88 aufweist, so daß der erste Hebel 54 die Anordnung aus dem Betätigungselement 28 und dem Betätigungselement 51 umgreift.

Die weitere Elektrode 40 ist in ihre proximale, in Fig. 2 dargestellte Position vorgespannt. Dies wird bei dem Ausführungsbeispiel dadurch erreicht, daß das Gelenk 58 der Hebelanordnung aus den Hebeln 54 und 56 unter Federspannung steht, die so wirkt, daß die Hebel 54 und 56 stets auseinandergedrückt werden (Darstellung mit unterbrochenen Linien in Fig. 5).

Während zuvor beschrieben wurde, daß das Betätigungselement 28 und das Betätigungselement 51 von der Handhabe 18 abnehmbar sind, ist auch der Schaft 12 mit der Handhabe 18 abnehmbar verbunden, wozu eine Kupplung 89 dient (vgl. Figuren 1 und 4), mittels der der Schaft 12 an dem Gehäuse 86 der Handhabe 18 verschraubt oder in der Art eines Bajonettverschlusses mit diesem verbunden ist.

Das Instrument 10 kann nun einerseits zum Präpariern von Gewebe mittels der Maulteile 14 und 16 verwendet werden, um beispielsweise Gewebeteile durch- oder abzutrennen. Dabei werden lediglich die Griffteile 20 und 22 betätigt, um die Maulteile 14 und 16 zu öffnen oder zu schließen. Tritt nun eine größere Gewebebrücke auf, die möglicherweise größere Gefäße enthält, kann mit dem Instrument 10 vor dem Durchtrennen einer solchen größeren Gewebebrücke das Gewebe vorher bipolar koaguliert werden. Dazu wird das Griffteil 52 betätigt, wodurch die weitere Elektrode 40 nach distal aus dem Schaft 12 herausgeschoben wird und mit den Maulteilen 14 bzw. 16 eine Elektrodenanordnung für eine bipolare Koagulation bildet. Durch Strombeaufschlagung der weiteren Elektrode 40 und der Maulteile 14 und 16 oder, wie zuvor beschrieben, nur eines der Maulteile 14 bzw. 16 kann das zwischen den Maulteilen 14 und 16 und der weiteren Elektrode 40 gegriffene Gewebe bipolar koaguliert werden. Anschließend kann nach Loslassen des Griffteils 52 bei zurückgezogener Elektrode 40 mittels den Maulteilen 14 und 16 weiter präpariert werden.

Das Instrument 10 ist, wie sich aus der vorausgehenden Beschreibung ergibt, in folgende Baugruppen zerlegbar: die Maulteile 14, 16 mit dem dazugehörigen Betätigungselement 28, die weitere Elektrode 40 mit dem dazugehörigen Betätigungselement 51, den Schaft 12 und die Handhabe 18.

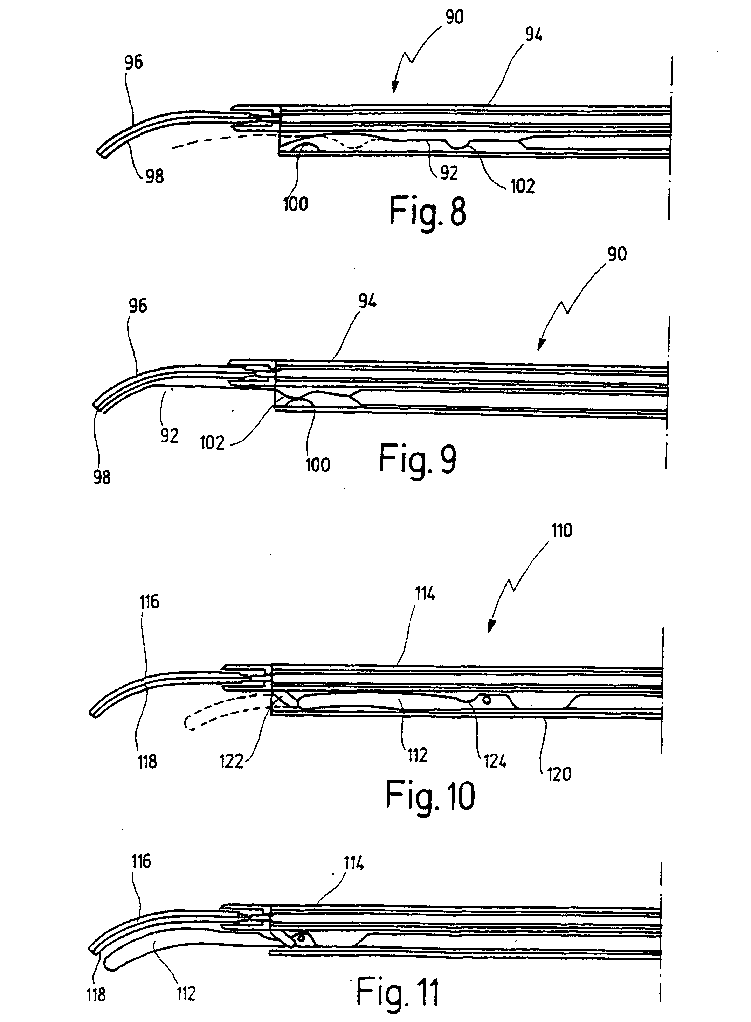

In Figuren 8 und 9 ist ein weiteres Ausführungsbeispiel eines Instruments 90 dargestellt, das sich von dem vorhergehenden Ausführungsbeispiel lediglich hinsichtlich der Ausgestaltung einer weiteren Elektrode 92 unterscheidet.

In Fig. 8 ist die weitere Elektrode 92 in ihrer in einem Schaft 94 zurückgezogenen proximalen Position dargestellt, während die weitere Elektrode 92 in Fig. 9 in ihrer nach distal verschobenen Position dargestellt ist, in der sie mit Maulteilen 96 und 98 eine bipolare Koagulationselektrodenanordnung bildet.

Die weitere Elektrode 92 ist im Unterschied zu der weiteren Elektrode 40 zumindest teilweise elastisch ausgebildet. Bei dem in Figuren 8 und 9 dargestellten Ausführungsbeispiel ist die weitere Elektrode 92 als flache Stahlblattfeder ausgebildet und somit insgesamt elastisch ausgestaltet. Am distalen Ende des Schafts 94 ist eine Anlaufschräge 100 ausgebildet. Die weitere Elektrode 92 weist im Bereich ihres proximalen Endes ebenfalls eine Anlaufschräge 102 auf.

Beim Vorschieben der weiteren Elektrode 92 beschreibt diese zunächst eine im wesentlichen in Längsrichtung des Schafts 94 verlaufende Bahnkurve, wie in Fig. 8 mit unterbrochenen Linien dargestellt ist. Beim noch weiteren Vorschieben der weiteren Elektrode 92 läuft dann die Anlaufschräge 102 auf die Anlaufschräge 100 auf, wodurch die weitere Elektrode 92 im letzten Abschnitt ihres Bewegungsweges eine Bahnkurve mit zu den Maulteilen 96 und 98 hin gerichteter Komponente beschreibt, so daß die weitere Elektrode 92 aktiv gegen die Maulteile 96, 98 gedrückt wird. Dadurch wird das zwischen den Maulteilen 96, 98 und der weiteren Elektrode 92 gegriffene Gewebe zum Koagulieren sicher eingeklemmt.

In Figuren 10 und 11 ist schließlich noch ein weiteres Ausführungsbeispiel eines Instruments 110 dargestellt, das sich hinsichtlich der zuvor beschriebenen Ausführungsbeispiele wiederum durch die Ausgestaltung einer weiteren Elektrode 112 unterscheidet. In Fig. 10 ist die weitere Elektrode 112 in ihrer in einem Schaft 114 des Instruments 110 zurückgezogenen proximalen Position und in Fig. 11 in ihrer nach distal verschobenen Position dargestellt. Das Instrument 110 weist wiederum Maulteile 116 und 118 auf, mit denen die weitere Elektrode 112 in der in Fig. 11 dargestellten distalen Position eine bipolare Koagulationsanordnung bildet. Es kann auch wieder vorgesehen sein, daß die weitere Elektrode 112 nur mit einem der Maulteile 116, 118 eine derartige Koagulationselektrodenanordnung bildet.

Im Unterschied zu dem vorhergehenden Ausführungsbeispiel ist die weitere Elektrode 112 teilweise elastisch ausgebildet, und zwar an einem proximalen Abschnitt 120. Im übrigen ist die weitere Elektrode 112 starr und massiv ausgebildet, und weist in etwa die Form eines Messers auf, jedoch ohne scharfe Schneidkante.

Am Schaft 114 ist wiederum eine Anlaufschräge 122 ausgebildet, die in Längsrichtung des Schafts 114 geschlitzt ausgebildet ist, wobei die weitere Elektrode 112 in dem Schlitz (nicht dargestellt) beim Vorschieben längs geführt wird.

Beim Vorschieben der weiteren Elektrode 112 läuft diese über die Anlaufschräge 122 und wird dabei zunächst von den Maulteilen 116, 118 abgespreizt, wie in Fig. 10 mit unterbrochenen Linien dargestellt ist, wodurch ein größerer Fangraum zwischen den Maulteilen 116, 118 und der weiteren Elektrode 112 erreicht wird.

Im proximalen Bereich weist die weitere Elektrode 112 eine Einbuchtung 124 auf. Sobald die Einbuchtung 124 die Anlaufschräge 122 beim Vorschieben der weiteren Elektrode 112 erreicht, federt die weitere Elektrode 112 elastisch gegen die Maulteile 116, 118. Die weitere Elektrode 112 beschreibt demnach beim Vorschieben eine Bahnkurve, die zunächst eine von den Maulteilen 116, 118 weg gerichtete Komponente und im letzten Teilstück des Bewegungsweges eine auf die Maulteile 116, 118 hin gerichtete Komponente aufweist.

## Patentansprüche

1. Medizinisches Instrument zum Präparieren von Gewebe im menschlichen oder tierischen Körper, mit einem langerstreckten Schaft (12; 94; 114), mit zwei relativ zueinander beweglichen Maulteilen (14, 16; 96, 98; 116, 118) am distalen Ende des Schafts (12; 94; 114), die schneidend und/oder fassend zusammenwirken, wobei zumindest eines der Maulteile (14, 16; 96, 98; 116, 118) als mit Hochfrequenzstrom beaufschlagbare Elektrode ausgebildet ist, **dadurch gekennzeichnet, daß** eine weitere mit Hochfrequenzstrom beaufschlagbare Elektrode (40; 92; 112) vorgesehen ist, die wahlweise aus einer zurückgezogenen proximalen Position in eine distale, den Maulteilen (14, 16; 96, 98; 116, 118) seitlich benachbarte Position verschiebbar ist, in der sie im Zusammenwirken mit dem zumindest einen als Elektrode ausgebildeten Maulteil (14, 16; 96, 98; 116, 118) den zweiten Pol einer Elektrodenanordnung zum Fassen und bipolaren Koagulieren von Gewebe zwischen der weiteren Elektrode (40; 92; 112) und den Maulteilen (14, 16; 96, 98; 116, 118) bildet.

2. Instrument nach Anspruch 1, **dadurch gekennzeichnet, daß** die Maulteile (14, 16; 96, 98; 116, 118) aus der Längsachse des Schafts (12; 94; 114) heraus gekrümmt sind, und daß die weitere Elektrode (40; 92; 112) auf der konkaven Seite der Maulteile (14, 16; 96, 98; 116, 118) angeordnet ist.

3. Instrument nach Anspruch 1 oder 2, **dadurch gekennzeichnet, daß** die weitere Elektrode (40; 92; 112) flächig ausgebildet ist und etwa die gleiche Breitenabmessung aufweist wie die beiden Maulteile (14, 16; 96, 98; 116, 118).

4. Instrument nach einem der Ansprüche 1 bis 3, **dadurch gekennzeichnet, daß** die weitere Elektrode (40; 92; 112) mit zumindest einem der Maulteile (14, 16; 96, 98; 116, 118) einen Fangraum (46) zum Greifen von Gewebe bildet.

5. Instrument nach einem der Ansprüche 1 bis 4, **dadurch gekennzeichnet, daß** die weitere Elektrode (40; 92; 112) in ihrer zurückgezogenen Position im Schaft (12; 94; 114) versenkt aufgenommen ist.

6. Instrument nach einem der Ansprüche 1 bis 5, **dadurch gekennzeichnet, daß** die weitere Elektrode (40) am distalen Ende auf der den Maulteilen (14, 16) zugewandten Seite abgeschrägt ist.

7. Instrument nach einem der Ansprüche 1 bis 5, **dadurch gekennzeichnet, daß** die weitere Elektrode (92; 112) zumindest teilweise elastisch ausgebildet ist und beim Vorschieben eine Bahnkurve beschreibt, die anfangs etwa in Längsrichtung des Schafts (94; 114) mit oder ohne geringfügig von den Maulteilen (96, 98; 116, 118) abgewandter Komponente und zu der distalen Position hin mit zu den Maulteilen (96, 98; 116, 118) hin gerichteter Komponente verläuft.

8. Instrument nach Anspruch 7, **dadurch gekennzeichnet, daß** am Schaft (94) eine Anlaufschräge (100) angeordnet ist, auf die eine an der weiteren Elektrode (92) ausgebildete Anlaufschräge (102) beim Vorschieben der Elektrode (92) aufläuft, wodurch die Elektrode (92) in der distalen Position gegen die Maulteile (96, 98) gedrückt wird.

9. Instrument nach Anspruch 7, **dadurch gekennzeichnet, daß** am Schaft (114) eine Anlaufschräge (122) angeordnet ist, so daß beim Vorschieben der weiteren Elektrode (112) diese von den Maulteilen (116, 118) zunächst abgespreizt wird und in der distalen Position elastisch gegen die Maulteile (116, 118) federt.

10. Instrument nach einem der Ansprüche 1 bis 9, **dadurch gekennzeichnet, daß** am proximalen Ende des Instruments (10) eine Handhabe (18) angeordnet ist, die zwei Griffteile (20, 22) zur Betätigung der Maulteile (14, 16) und ein weiteres Griffteil (52) zur Betätigung der weiteren Elektrode (40) aufweist, wobei die Griffteile (20, 22, 52) eine mit einer Hand bedienbare Griffanordnung bilden.

11. Instrument nach Anspruch 10, **dadurch gekennzeichnet, daß** das weitere Griffteil (52) über eine Hebelanordnung (54, 56) mit der weiteren Elektrode (40) derart verbunden ist, daß durch Ziehen des Griffteils (52) die weitere Elektrode (40) aus ihrer proximalen in ihre distale Position geschoben wird.

12. Instrument nach Anspruch 10 oder 11, **dadurch gekennzeichnet, daß** die weitere Elektrode (40) in ihre proximale Position vorgespannt ist, in die sie nach Loslassen des weiteren Griffteils (52) selbsttätig zurückkehrt.

13. Instrument nach einem der Ansprüche 10 bis 12, **dadurch gekennzeichnet, daß** die weitere Elektrode (40) über ein axial beweglich in dem Schaft (12) aufgenommenes Betätigungselement (51) mit dem weiteren Griffteil (52) verbunden ist, wobei das Betätigungselement (51) weiterhin über einen Schleifkontakt (78) mit einer Hochfrequenzstrom-Zuführung (76) verbunden ist.

14. Instrument nach Anspruch 13, **dadurch gekennzeichnet, daß** der Schleifkontakt (78) eine Stromdurchführung auf die weitere Elektrode (40) erst zuläßt, wenn die Elektrode (40) in die distale Position oder nahezu in die distale Position vorgeschoben ist.

15. Instrument nach Anspruch 13 oder 14, **dadurch gekennzeichnet, daß** das Betätigungselement (51) mit dem weiteren Griffteil (52) abnehmbar, vorzugsweise durch Verrasten verbunden ist.

16. Instrument nach einem der Ansprüche 10 bis 15, **dadurch gekennzeichnet, daß** die Maulteile (14, 16) über ein axial beweglich in dem Schaft (12) aufgenommenes Betätigungselement (28) abnehmbar mit den Griffteilen (20, 22) verbunden sind.

17. Instrument nach Anspruch 16, **dadurch gekennzeichnet, daß** die Maulteile (14, 16) über einen Bajonettverschluß mit dem distalen Ende des Schafts (12) verbunden sind.

18. Instrument nach einem der Ansprüche 10 bis 17, **dadurch gekennzeichnet, daß** es in die Baugruppen aus den Maulteilen (14, 16) mit dem mit diesen verbundenen Betätigungselement (28), der weiteren Elektrode (40) mit dem weiteren Betätigungselement (51), der Handhabe (18) und dem Schaft (12) zerlegbar ist.

## Claims

1. A medical instrument for dissecting tissue in the human or animal body, comprising an elongated shaft (12; 94; 114), two jaw parts (14, 16; 96, 98; 116, 118) at the distal end of the shaft (12; 94; 114) that are movable relative to one another and that coact cuttingly and/or graspingly, at least one of the jaw parts (14, 16; 96, 98; 116, 118) being configured as an electrode to which high-frequency current can be applied, **characterized in that** a further electrode (40; 92; 112) that can have high-frequency current applied to it is provided, and can be displaced selectably from a retracted proximal position into a distal position adjacent to the jaw parts (14, 16; 96, 98; 116, 118) in which it forms, in coaction with the at least one jaw part (14, 16; 96, 98; 116, 118) configured as an electrode, the second pole of an electrode arrangement for grasping and bipolar coagulating of tissue between the further electrode (40; 92; 112) and the jaw parts (14, 16; 96, 98; 116, 118).

2. The instrument of claim 1, **characterized in that** the jaw parts (14, 16; 96, 98; 116, 118) are curved out of the longitudinal axis of the shaft (12; 94; 114), and the further electrode (40; 92; 112) is arranged on the concave side of the jaw parts (14, 16; 96, 98; 116, 118).

3. The instrument of claim 1 or 2, **characterized in that** the further electrode (40; 92; 112) is of planar configuration and has approximately the same width dimension as the two jaw parts (14, 16; 96, 98; 116, 118).

4. The instrument of anyone of claims 1 through 3, **characterized in that** the further electrode (40; 92; 112) forms, with at least one of the jaw parts (14, 16; 96, 98; 116, 118), a catching space (46) for grasping tissue.

5. The instrument of anyone of claims 1 through 4, **characterized in that** the further electrode (40; 92; 112), in its retracted position, is received in recessed fashion in the shaft (12; 94; 114).

6. The instrument of anyone of claims 1 through 5, **characterized in that** the further electrode (40) is beveled at the distal end on the side facing toward the jaw parts (14, 16).

7. The instrument of anyone of claims 1 through 5, **characterized in that** the further electrode (92; 112) is of at least partially flexible configuration and describes, when advanced, a trajectory that initially extends approximately in the longitudinal direction of the shaft (94; 114) with or without a component turned slightly away from the jaw parts (96, 98; 116, 118), and toward the distal position extends with a component directed toward the jaw parts (96, 98; 116, 118).

8. The instrument of claim 7, **characterized in that** there is arranged on the shaft (94) a cam bevel (100) onto which a cam bevel (102) configured on the further electrode (92) runs as the electrode (92) is advanced, thus pressing the electrode (92) toward the jaw parts (96, 98) in the distal position.

9. The instrument of claim 7, **characterized in that** a cam bevel (122) is arranged on the shaft (114) so that as the further electrode (112) is advanced, it is first spread away from the jaw parts (116, 118) and, in the distal position, deflects elastically toward the jaw parts (116, 118).

10. The instrument of anyone of claims 1 through 9, **characterized in that** there is arranged at the proximal end of the instrument (10) a handle (18) that has two grip elements (20, 22) for actuation of the jaw parts (14, 16) and a further grip element (52) for actuation of the further electrode (40), the grip elements (20, 22, 52) forming a grip arrangement operable with one hand.

11. The instrument of claim 10, **characterized in that** the further grip element (52) is joined via a lever arrangement (54, 56) to the further electrode (40) in such a way that by pulling the further grip element (52), the further electrode (40) is slid from its proximal into its distal position.

12. The instrument of claim 10 or 11, **characterized in that** the further electrode (40) is preloaded into its proximal position, into which it automatically returns after the further grip element (52) is released.

13. The instrument of anyone of claims 10 through 12, **characterized in that** the further electrode (40) is joined to the further grip element (52) via an actuation element (51) received in axially movable fashion in the shaft (12), the actuation element (51) furthermore being joined via a wiper contact (78) to a high-frequency current lead-in (76).

14. The instrument of claim 13, **characterized in that** the wiper contact (78) allows the passage of current to the further electrode (40) only when the electrode (40) has been advanced into or almost into the distal position.

15. The instrument of claim 13 or 14, **characterized in that** the actuation element (51) is joined to the further grip element (52) removably, preferably by snap-locking.

16. The instrument of anyone of claims 10 through 15, **characterized in that** the jaw parts (14, 16) are removably joined to the grip elements (20, 22) by way of an actuation element (28) received in axially movable fashion in the shaft (12).

17. The instrument of claim 16, **characterized in that** the jaw parts (14, 16) are joined to the distal end of the shaft (12) via a bayonet closure.

18. The instrument of anyone of claims 10 through 17, **characterized in that** it can be disassembled into the subassemblies made up of the jaw parts (14, 16) with the actuation element (28) joined thereto, the further electrode (40) with the further actuation element (51), the handle (18), and the shaft (12).

## Revendications

1. Instrument médical pour préparer des tissus de corps humains ou animaux, comportant une tige (12 ; 94 ; 114) allongée, deux parties de mâchoire (14, 16 ; 96, 98 ; 116, 118) mobiles l'une par rapport à l'autre à l'extrémité distale de la tige (12 ; 94 ; 114) qui coopèrent par coupe et/ou prise, l'une au moins des parties de mâchoire (14, 16 ; 96, 98 ; 116, 118) étant réalisée sous la forme d'une électrode pouvant être soumise à un courant à haute fréquence, **caractérisé en ce qu'**il est prévu une autre électrode (40 ; 92 ; 112) pouvant être soumise à un courant à haute fréquence, qui peut coulisser au choix depuis une position proximale en retrait à une position distale, voisine latéralement des parties de mâchoire (14, 16 ; 96, 98 ; 116, 118) dans laquelle elle forme en coopération avec au moins la partie de mâchoire (14, 16 ; 96, 98 ; 116, 118) réalisée en tant qu'électrode, le deuxième pôle d'un agencement d'électrodes pour saisir et pour coaguler de manière bipolaire des tissus entre l'autre électrode (40 ; 92 ; 112) et les parties de mâchoire (14, 16 ; 96, 98 ; 116, 118).

2. Instrument selon la revendication 1, **caractérisé en ce que** les parties de mâchoire (14, 16 ; 96, 98 ; 116, 118) sont courbées à l'extérieur de l'axe longitudinal de la tige (12 ; 94 ; 114) et **en ce que** l'autre électrode (40 ; 92 ; 112) est disposée sur le côté concave des parties de mâchoire (14, 16 ; 96, 98 ; 1 16, 118)

3. Instrument selon la revendication 1 ou 2, **caractérisé en ce que** l'autre électrode (40 ; 92 ; 112) est réalisée plate et présente à peu près la même dimension en largeur que les deux parties de mâchoire (14, 16 ; 96, 98 ; 116, 118).

4. Instrument selon l'une des revendications 1 à 3, **caractérisé en ce que** l'autre électrode (40 ; 92 ; 112) forme avec au moins l'une des parties de mâchoire (14, 16 ; 96, 98 ; 116, 118) un espace de retenue (46) pour saisir des tissus.

5. Instrument selon l'une des revendications 1 à 4, **caractérisé en ce que** l'autre électrode (49 ; 92 ; 112), dans sa position en retrait, est reçue encastrée dans la tige (12 ; 94 ; 114).

6. Instrument selon l'une des revendications 1 à 5, **caractérisé en ce que** l'autre électrode (40) est chanfreinée à l'extrémité distale sur le côté tourné vers les parties de mâchoire (14, 16).

7. Instrument selon l'une des revendications 1 à 5, **caractérisé en ce que** l'autre électrode (92 ; 112) est réalisée au moins en partie élastique et décrit lors de son avance une trajectoire courbe qui au début s'étend à peu près dans la direction longitudinale de la tige (94 ; 114) avec ou sans une composante légèrement tournée à l'opposé des parties de mâchoire (96, 98 ; 116, 118) et, vers la position distale, avec une composante orientée vers les parties de mâchoire (96, 98 ; 116, 118).

8. Instrument selon la revendication 7, **caractérisé en ce que** sur la tige (94) est disposée une surface oblique de butée (100) sur laquelle vient buter une surface oblique de butée (102) réalisée sur l'autre électrode (92), lors de l'avance de l'électrode (92), ce qui fait que l'électrode (92) est pressée en position distale contre les parties de mâchoire (96, 98).

9. Instrument selon la revendication 7, **caractérisé en ce que** sur la tige (114) est disposée une surface oblique de butée (122), de sorte que lors de l'avance de l'autre électrode (112), celle-ci est d'abord écartée des parties de mâchoire (116, 118) et, en position distale, elle fait ressort élastiquement contre les parties de mâchoire (116, 118).

10. Instrument selon l'une des revendications 1 à 9, **caractérisé en ce qu'**à l'extrémité proximale de l'instrument (10) est disposée une manette (18) qui comporte deux parties de poignée (20, 22) pour l'actionnement des parties de mâchoire (14, 16) et une autre partie de poignée (52) pour l'actionnement de l'autre électrode (40), les parties de poignée (20, 22 ; 52) formant un dispositif de poignée pouvant être commandé d'une main.

11. Instrument selon la revendication 10, **caractérisé en ce que** l'autre partie de poignée (52) est reliée à l'autre électrode (40) par un agencement de levier (54, 56), de manière que lorsque l'on tire la partie de poignée (52), l'autre électrode (40) est poussée de sa position proximale à sa position distale.

12. Instrument selon la revendication 10 ou 11, **caractérisé en ce que** l'autre électrode (40) est précontrainte dans sa position proximale dans laquelle elle se replace automatiquement lorsque l'on relâche l'autre partie de poignée (52).

13. Instrument selon l'une des revendications 10 à 12, **caractérisé en ce que** l'autre électrode (40) est reliée, par un élément d'actionnement (51) reçu axialement mobile dans la tige (12), à l'autre partie de poignée (52), l'élément d'actionnement (51) étant relié en outre, par un contact frottant (78), à une amenée de courant à haute fréquence (76).

14. Instrument selon la revendication 13, **caractérisé en ce que** le contact frottant (78) n'autorise d'abord un passage du courant que si l'électrode (40) est poussée dans la position distale ou presque dans la position distale.

15. Instrument selon la revendication 13 ou 14, **caractérisé en ce que** l'élément d'actionnement (51) est relié à l'autre partie de poignée (52), de manière amovible, de préférence par encliquetage.

16. Instrument selon l'une des revendications 10 à 15, **caractérisé en ce que** les parties de mâchoire (14, 16) sont reliées aux parties de poignée (20, 22), de manière amovible, par un élément d'actionnement (28) reçu axialement mobile dans la tige (12).

17. Instrument selon la revendication 16, **caractérisé en ce que** les parties de mâchoire (14, 16) sont reliées à l'extrémité distale de la tige (12), par une fermeture à baïonnette.

18. Instrument selon l'une des revendications 10 à 17, **caractérisé en ce qu'**il peut être démonté dans les ensembles composés des parties de mâchoire (14, 16) avec l'élément d'actionnement (28) de l'autre électrode (40) avec l'autre élément d'actionnement (51), de la manette (18) et de la tige (12).
